# EUROPEAN PATENT APPLICATION

(11) **EP 3 673 954 A1**
(43) Date of publication of application: **01.07.2020**
(21) Application number: 19208575.1
(22) Date of filing: 12.11.2019
(51) Int. Cl.: A61N 5/06

(54) **DEVICE AND METHOD FOR INDUCING IMPROVEMENT OF CEREBRAL CIRCULATION**

(30) Priority: 27.12.2018 KR 20180170760
(71) Applicant: Color Seven.Co., Ltd, Seoul (KR)
(72) Inventor: KIM, Nam Gyun, Seoul (KR); PARK, Kyoung Jun, Seoul (KR)
(74) Representative: Danubia Patent & Law Office LLC

(57) **Abstract**

The present disclosure relates to a device and method for inducing improvement of cerebral circulation by using light energy, i.e., photo-innervated neuro-stimulation (PINS), and stimulating nerve endings distributed in the epidermis or dermis located below a skin surface in an area corresponding to the carotid artery or an area corresponding to the vertebral artery of the cervix so that blood vessels (vascular muscles) in the brain are relaxed.

The present disclosure can be utilized in treating or preventing vascular dementia and Alzheimer's disease, which are caused by the lack of cerebral circulation, and various other brain-related diseases, such as stroke, migraine, depression, and Parkinson's disease, and improving learning ability, memory, and concentration.

## Description

### CROSS-REFERENCE TO RELATED APPLICATION

This application claims priority to and the benefit of Korean Patent Application No. 10-2018-0170760, filed on December 27, 2018, the disclosure of which is incorporated herein by reference in its entirety.

### BACKGROUND

### 1. Field of the Invention

The present disclosure relates to a device and method for inducing improvement of cerebral circulation, and more particularly to a device and method for inducing improvement of cerebral circulation by using light energy, i.e., photo-innervated neuro-stimulation (PINS), and stimulating nerve endings distributed in the epidermis or dermis located below a skin surface in a specific region of a neck or a back portion of the neck so that blood vessels (vascular muscles) in the brain are relaxed.

### 2. Discussion of Related Art

Blood can be said to control the metabolism of the whole body. Blood flows throughout the human body and takes charge of all sorts of tasks necessary to sustain life. Blood serves to carry oxygen from the lung to each tissue or cell, obtain nutrients from the digestive organs, e.g., the stomach or intestine, and deliver the right nutrients in the right places and also serves to send oxygen and nutrients used up in the tissues and cells to excretory organs. The flow of healthy blood is required for all of the above functions to be performed smoothly. Blood vessels consist of vascular muscle cells.

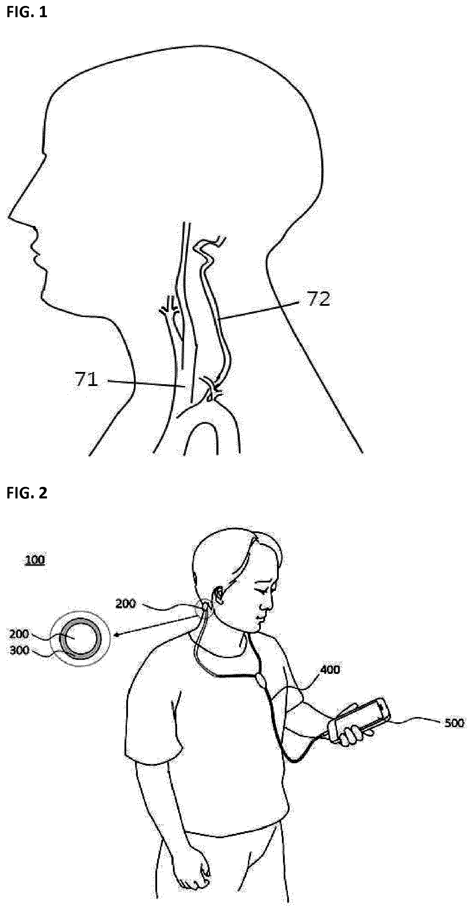

As illustrated in FIG. 1, the brain receives blood and oxygen through a carotid artery (71) and a vertebral artery (72).

The carotid artery is a branch of the aorta. The left and right carotid arteries originate from the aortic arch, rise along the outer side of the larynx, and enter the facial bones and cranial bones. The carotid artery is branched into the internal carotid artery and the external carotid artery in the vicinity of the thyroid. The external carotid artery sends arterial blood to the head and face while the internal carotid artery sends arterial blood to each part of the brain.

The vertebral artery is one artery distributed in the brain. The vertebral artery is divided from the base of the subclavian arteries at both sides, ascends into the transverse foramen of the sixth cervical vertebra, passes through another transverse foramen located sequentially above the transverse foramen of the sixth cervical vertebra, moves further upward, is bent in a dorsomedial direction from an upper surface of the atlas (the first cervical vertebra), and, in a portion between the occipital bone and the vertebral artery, pierces the dura mater and the arachnoid, enters the subarachnoid cavity, and enters the cranial cavity through the foramen magnum.

Particularly, about 20% of the blood from the heart is required to be supplied to the brain to allow the brain to function smoothly. When the oxygen in the blood is insufficient, the brain itself supplies more blood to the brain to fill the insufficient oxygen. As a result of such an activity, the intracranial pressure is increased. It has been reported that sudden increase in intracranial pressure causes headaches and dizziness due to poor blood circulation and poor oxygen supply.

In many cases, due to the narrowing of the carotid artery or the vertebral artery which supplies energy and oxygen required for the brain, blood circulation becomes poor, and thus cerebral circulation is decreased. When memory decreases and headaches occur due to the decreased cerebral circulation, work efficiency or learning ability is decreased, which leads to stress again, resulting in a vicious cycle. Cerebral circulation problems are very important because the problems may not only simply cause headaches or dizziness but also cause diseases such as cognitive impairment, vascular dementia, Alzheimer's disease, and Parkinson's disease.

Symptoms such as cognitive impairment, vascular dementia, Alzheimer's disease, and Parkinson's disease are known as symptoms caused by lack of supply of oxygen and nutrients to neurons of the brain that occurs due to poor cerebral circulation for a long period that is caused by problems in the contraction and relaxation of smooth muscles of blood vessels in the brain. Also, it is known that diseases such as Alzheimer's disease occur due to the accumulation of amyloid beta (A-beta) plaque that occurs as, due to poor cerebral circulation, neuroglial cells responsible for the clearance system of the brain are damaged or functions thereof are impaired, and wastes such as metabolites of various cells that are generated every day are not properly discharged every day.

Numerous new drug development studies have been conducted to treat or alleviate symptoms such as cognitive impairment and dementia, but the symptoms have not been treated well or alleviated by drugs. Also, attempts to alleviate the symptoms by using alternative therapies such as acupuncture, moxibustion, and thermotherapy or stimulating brain cells using electric or magnetic fields and far infrared rays have been made but are known to have little effect. Side effects of drugs have been raised as serious problems of treatment by drugs, and the possibilities of causing injuries or burns to the human body and causing hygiene problems when one neglects to disinfect wounds due to the injuries or burns have been raised as problems of alternative therapies such as acupuncture and moxibustion. In addition, regarding treatment devices using electric or magnetic fields, inconvenience in use due to large sizes of the treatment devices or difficulties in accurately determining locations of points of stimulation and little effect in alleviating the symptoms have been pointed out as problems. Also, regarding nerve stimulators which are partially inserted into the human body for use, despite being relatively effective, high costs for the device and surgery for inserting the device into the human body have been pointed out as problems.

Meanwhile, a light irradiator that stimulates the brain using the low-level light therapy (LLLT) may also be used. However, since, due to a problem that light has to pass through the skull, light having a very high intensity is used as light having a long wavelength which belongs to the far-infrared spectrum, the light causes a temperature rise of the brain such that a cooling device for preventing the temperature rise may be required or the light output of the light irradiator may increase, causing a natural increase in the power consumption and an increase in the weight or size of equipment. Thus, the light irradiator may be inconvenient to use.

In recent years, it has been reported that cerebral circulation is closely related to improvement of learning ability, memory, and concentration and treatment or prevention of cognitive impairment, Alzheimer's disease, and Parkinson's disease. Accordingly, there is an urgent need for technological development on new devices and methods that induce improvement of cerebral circulation more easily and conveniently.

The inventors of the present disclosure hold, as patents related to a light therapy device, Korean Patent Publication No. 10-2012-0131316, Korean Patent Registration No. 10-1225135, Korean Patent Publication No. 10-2016-0049097, Korean Patent Publication No. 10-2016-0054423, etc.

The related art, Korean Patent Registration No. 10-1009462, relates to a near-infrared light therapy device in the form of a helmet that irradiates the inside of the skull and the cranial base region with near infrared light for treatment of dementia and the like. The near-infrared light therapy device is inconvenient to carry and has to be custom-made or each user because the size of the head is different for each user.

### [Related Art Document]

(Patent Document 1) Korean Patent Registration No. 10-1009462

### SUMMARY OF THE INVENTION

The present disclosure is directed to providing a device and method for inducing improvement of cerebral circulation by using photo-innervated neuro-stimulation (PINS) to stimulate, for a predetermined time, nerve endings distributed in the epidermis or dermis located below a skin surface in a specific region of a neck or a back portion of the neck and inducing substances such as nitric oxide to be secreted from nitrergic nerve terminals connected to the stimulated nerve endings through the peripheral nervous system so that the secreted substances relax cerebral vessels in contact with the nitrergic nerve terminals and induce the improvement of cerebral circulation.

A device for inducing improvement of cerebral circulation according to one aspect of the present disclosure includes: a light irradiator which includes a light source and irradiates, with light, an area corresponding to a carotid artery or an area corresponding to a vertebral artery, which is a skin surface of a cervix in an area where the carotid artery or the vertebral artery which supplies blood and oxygen to the brain is located, according to a light irradiation control signal; and a light irradiation control device which generates the light irradiation control signal and transmits the light irradiation control signal to the light irradiator according to setting data including a light irradiation time.

The light irradiator may stimulate nerve endings distributed in the epidermis or dermis located below the skin surface in the area corresponding to the carotid artery or the area corresponding to the vertebral artery by irradiating the nerve endings with light for a predetermined light irradiation time and induce a substance containing nitric oxide to be secreted from nitrergic nerve terminals connected to the stimulated nerve endings through the nervous system, and the secreted substance may relax cerebral vessels and lymphatic vessels which are in contact with the nitrergic nerve terminals and induce the improvement of cerebral circulation.

The light irradiator may be controlled, by a light irradiation control device, to output visible light for suppressing accumulation of amyloid beta plaque by, as the cerebral vessels and the lymphatic vessels which are in contact with the nitrergic nerve terminals are relaxed and the improvement of cerebral circulation is induced, improving supply of oxygen and nutrients to cells, such as astrocytes, that are damaged which are responsible for functioning of the clearance system of the brain and gradually recovering impaired functions of the clearance system of the brain and to emit the visible light for a set light irradiation time and automatically end emission of the visible light when the set light irradiation time ends.

The light irradiation control device may wirelessly transmit the light irradiation control signal to the light irradiator, and, in this case, the light irradiator may have a battery embedded therein. Alternatively, the light irradiation control device may transmit the light irradiation control signal to the light irradiator via a wire, and, in this case, the light irradiator may be driven by power transmitted from the light irradiation control device.

The area corresponding to the carotid artery may be at a location of Large Intestine 18 (LI 18) point in the cervix of a human or an animal, and the area corresponding to the vertebral artery may be at a location of Urinary Bladder 10 (UB 10) point or Gallbladder 20 (GB 20) point in the cervix of a human or an animal.

When, along left and right sides of a midline or an Adam's apple at the front of the cervix, a straight-line distance from the midline or the Adam's apple to a line vertically extending downward from an earlobe is referred to as "front midline-earlobe distance," the light irradiator may be mounted between a 1/2 point of the front midline-earlobe distance from the midline or the Adam's apple and a 2/3 point of the front midline-earlobe distance from the midline or the Adam's apple.

When, along left and right sides of a midline at the back of the cervix, a straight-line distance from the midline to a line vertically extending downward from an earlobe is referred to as "back midline-earlobe distance," the light irradiator may be mounted between a 1/2 point of the back midline-earlobe distance from the midline and a 2/3 point of the back midline-earlobe distance from the midline.

The light source may be one of a light emitting diode (LED), an organic light emitting diode (OLED), or a micro LED or a combination of one or more thereof. The light irradiator may be configured to emit light whose peak wavelength is in a range of 590 nm to 620 nm and whose intensity is in a range of 1 mW/cm² to 5 mW/cm².

The light irradiator may further include an attachment tool which allows the light irradiator to be mounted on an animal or a human to irradiate the area corresponding to the carotid artery or the area corresponding to the vertebral artery with light, and the attachment tool may be one of a double-sided tape, a suction tool, a headgear, a necklace, and a neck strap.

The light irradiator may be configured to output light for 10 minutes to 20 minutes to stimulate the area corresponding to the carotid artery or the area corresponding to the vertebral artery one time, the light irradiation control device may be one of a smartphone, a personal digital assistant (PDA), a laptop, an Android phone, an iPhone, a smartwatch, and a computer, and the light irradiator may further include a light filter.

When a periodical treatment mode switch included in the light irradiation control device is selected, the light irradiation control device may generate, at every predetermined time interval, a light irradiation control signal, which allows the light irradiator to output light for a predetermined light irradiation time, and transmit the light irradiation control signal to the light irradiator.

When the attachment tool is one of the headgear, the neck strap, and the necklace, the light irradiation control device or the light irradiator may be embedded in the attachment tool.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a schematic diagram for describing a carotid artery and a vertebral artery;
FIG. 2 is a view illustrating a use state of a device for inducing improvement of cerebral circulation according to an embodiment of the present disclosure;
FIG. 3 is a view illustrating a use state of the device for inducing improvement of cerebral circulation according to another embodiment of the present disclosure;
FIG. 4 is a view for describing positions at which a light irradiator according to the present disclosure is adhered or brought close to the cervix of a human to irradiate visible light;
FIG. 5 is a view for describing positions at which the light irradiator according to the present disclosure is adhered or brought close to the cervix (neck) of an animal to irradiate visible light;
FIG. 6 illustrates an example of a light irradiator (200) according to the present disclosure;
FIG. 7 is a block diagram illustrating a configuration of a device for inducing improvement of cerebral circulation according to the present disclosure;
FIG. 8 illustrates an example of a graph showing results of imaging cerebral vessels before and after light stimulation applied to an animal using a device and method for inducing improvement of cerebral circulation according to the present disclosure;
FIG. 9 illustrates graphs showing degrees of relaxation of blood vessels for each vessel diameter length before and after light stimulation experimented on an animal using the device and method for inducing improvement of cerebral circulation according to the present disclosure; and
FIG. 10 illustrates an experimental protocol in which, using the device and method for inducing improvement of cerebral circulation according to the present disclosure, cerebral vessels are relaxed to induce improvement of cerebral circulation in a normal person and illustrates a result of conducting an experiment according to the experimental protocol.

### DETAILED DESCRIPTION OF EXEMPLARY EMBODIMENTS

Hereinafter, a device and method for inducing improvement of cerebral circulation according to the present disclosure will be described in more detail with reference to the accompanying drawings.

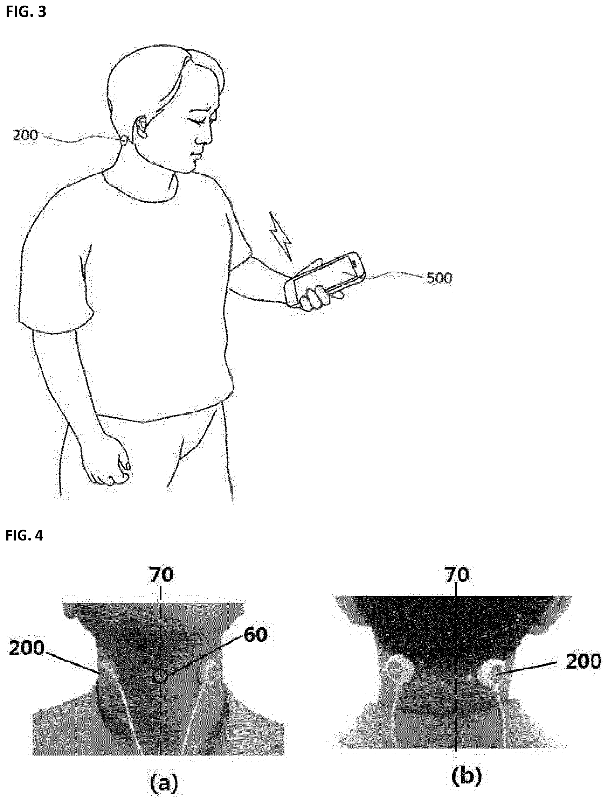

FIG. 2 is a view illustrating a use state of the device for inducing improvement of cerebral circulation according to an embodiment of the present disclosure, and FIG. 3 is a view illustrating a use state of the device for inducing improvement of cerebral circulation according to another embodiment of the present disclosure.

A device 100 for inducing improvement of cerebral circulation is a device that irradiates, with light, a specific portion of a neck where blood vessels (carotid arteries or vertebral arteries) which supply blood and oxygen to the brain are located, relaxes the blood vessels, i.e., vascular muscles, and improves (facilitates, activates) the supply of blood and oxygen to the brain. The device 100 for inducing improvement of cerebral circulation includes a light irradiator 200, an attachment tool 300, and a light irradiation control device 500.

FIG. 2 illustrates an example of the device 100 for inducing improvement of cerebral circulation in which the light irradiator 200 and the light irradiation control device 500 are connected to each other via a wire. The device 100 for inducing improvement of cerebral circulation further includes a cable 400 which connects the light irradiator 200 and the light irradiation control device 500 to each other. That is, the device 100 for inducing improvement of cerebral circulation illustrated in FIG. 2 uses a double-sided tape as the attachment tool 300. In this case, the light irradiator 200 is connected to the light irradiation control device 500 through the cable 400, receives power from the light irradiation control device 500, and receives a light irradiation control signal from the light irradiation control device 500. For example, the light irradiator 200 may be adhered to a skin surface in a specific region of an animal or human body using a double-sided tape, and a user may carry the light irradiation control device 500 connected to the light irradiator 200 through the cable 400 and perform light irradiation by himself or herself.

FIG. 3 illustrates an example of the device for inducing improvement of cerebral circulation in which the light irradiator 200 and the light irradiation control device 500 are wirelessly connected to each other. In this case, the light irradiator 200 and the light irradiation control device 500 each further include a wireless transmission/reception unit.

In the device for inducing improvement of cerebral circulation illustrated in FIG. 3, when a battery and a wireless transmission/reception device are further embedded in the light irradiator 200, the light irradiator 200 may be operated separately from the light irradiation control device 500 and thus wirelessly control light irradiation.

The light irradiator 200 is a device that emits light according to a light irradiation control signal received from the light irradiation control device 500. The light irradiator 200 is mounted on a human or animal body using the attachment tool 300. The light irradiator 200 is configured to emit light whose peak wavelength is in a range of 590 nm to 620 nm and whose intensity is in a range of 1 mW/cm² to 5 mW/cm². A light source 270 embedded in the light irradiator 200 may be one of a light emitting diode (LED), an organic light emitting diode (OLED), or a micro LED or a combination of one or more thereof.

The attachment tool 300 is a device that allows the light irradiator 200 to be attached or brought close to the cervix (neck). The attachment tool 300 may be in the form of a double-sided tape, a suction tool, a headgear worn over the head or ears, a necklace (a neck strap worn around the neck), or the like.

The light irradiation control device 500 is a device that controls light irradiation by the light irradiator 200. The light irradiation control device 500 may include a display unit 530, an arithmetic processing unit 550 (a microprocessor or a light irradiation control circuit), a wireless transmission/reception unit 510, a power supply device 490, a key input unit (an on/off switch (a start button and an end button) or the like), a light irradiation operation indicator lamp (e.g., an LED) (not illustrated), an alarm device (not illustrated) and the like. The light irradiation control device 500 may be a microprocessor, a microcontroller, a computer, or a portable personal terminal (smart communication device). According to circumstances, the light irradiation control device 500 may include a microprocessor, a microcontroller, and a smart communication device. The portable personal terminal (smart communication device) may be a smart communication device such as a smartphone, a personal digital assistant (PDA), a laptop, an Android phone, an iPhone, or a smartwatch, have a predetermined application installed therein, and be carried by a user and allow the user to receive light therapy anytime and anywhere by controlling the light irradiator 200 by himself or herself.

The light irradiation control device 500 may also be manufactured for use in hospitals. The light irradiation control device 500 may be manufactured to be large and sturdy to be installed in hospitals and may allow an animal or a patient, who visits the hospital, to receive light therapy by utilizing the light irradiator 200.

When the attachment tool 300 is in the form of a headgear or a headband worn over the head or ears, the light irradiation control device 500 may be in the form of a microprocessor or a microcontroller and embedded and installed in a portion of a body of the headgear or the headband. In this case, the light irradiation control device 500 may be connected to the smart communication device via a wire or wirelessly, and the light irradiation control device 500 may be controlled using the smart communication device to control the light irradiation by the light irradiator 200.

When the attachment tool 300 is in the form of a necklace (neck strap), the light irradiation control device 500 may be embedded and installed in a portion of a body of the necklace (neck strap). In this case, the light irradiation control device 500 may be connected to the smart communication device via a wire or wirelessly, and the light irradiation control device 500 may be controlled using the smart communication device to control the light irradiation by the light irradiator 200.

In a state in which the light irradiator 200, which has a light filter and a light source, which is configured to emit visible light in the visible spectrum, embedded therein and emits the visible light emitted from the light source through one surface, is directly adhered or brought close to a skin area in a specific region of a neck of an animal or human body by the attachment tool 300, the light irradiator 200 is controlled to output visible light for suppressing accumulation of amyloid beta plaque by stimulating nerve endings distributed in the epidermis or dermis located below the skin surface in the corresponding region for a predetermined time, inducing a substance such as nitric oxide to be secreted from nitrergic nerve terminals connected to the stimulated nerve endings through the nervous system, using the secreted substance, relaxing cerebral vessels and lymphatic vessels, which are in contact with the nitrergic nerve terminals, to induce the improvement of cerebral circulation and lymphatic circulation, and improving supply of oxygen and nutrients to cells, such as astrocytes, that are damaged which are responsible for functioning of the clearance system of the brain (that is, astrocytes that are damaged which are responsible for functioning of the clearance system of the brain) to gradually recover impaired functions of the clearance system of the brain. Also, the light irradiator 200 is controlled to emit the corresponding visible light for a set light irradiation time and automatically end emission of the visible light when the set light irradiation time ends.

Generally, astrocytes are also referred to as "scavenger cells."

In a state in which the light irradiator 200 is directly adhered or brought close to a skin surface located at a portion of each of left and right vertebral arteries or a portion of each of left and right carotid arteries at a back portion of the neck of the animal or human body by the attachment tool 300, the light irradiation control device 500 suppresses the accumulation of amyloid beta plaque by stimulating nerve endings distributed in the epidermis or dermis located below the skin surfaces at the locations of the vertebral arteries or the carotid arteries for a predetermined time using the visible light emitted from the light irradiator 200 to induce secretion of a substance, such as nitric oxide produced by neuronal nitric oxide synthase (nNOS), from nitrergic nerve terminals around cerebral vessels which are connected to the stimulated nerve endings through peripheral nerves, using the secreted substance, relaxing cerebral vessels and lymphatic vessels, which are in contact with the nitrergic nerve terminals, to repeatedly induce the improvement of cerebral circulation and lymphatic circulation, and improving supply of oxygen and nutrients to cells, such as astrocytes, that are damaged which are responsible for functioning of the clearance system of the brain to gradually recover impaired functions of the clearance system of the brain.

FIG. 4 is a view for describing positions at which a light irradiator according to the present disclosure is adhered or brought close to the cervix (neck) of a human to irradiate visible light.

FIG. 4A illustrates the front of the subject and illustrates a state in which the light irradiator 200 is mounted on each area corresponding to a carotid artery of the cervix (neck) of the subject. For example, along left and right sides of a midline 70 or an Adam's apple 60, a straight-line distance from the midline 70 or the Adam's apple 60 to a line vertically extending downward from an earlobe will be referred to as "front midline-earlobe distance" for convenience of description in the present disclosure. The front midline-earlobe distance may be about 1/4 of the neck circumference. Then, to mount the light irradiator 200 on the area corresponding to the carotid artery, the light irradiator 200 may be mounted between a 1/2 point of the front midline-earlobe distance from the midline 70 or the Adam's apple 60 and a 2/3 point of the front midline-earlobe distance from the midline 70 or the Adam's apple 60.

Alternatively, when the light irradiator 200 is mounted on the area corresponding to the carotid artery of the cervix (neck) of the subject as in FIG. 4A, the light irradiator 200 may be mounted on Large Intestine 18 (LI 18) point on the front of the neck (the front of the cervix) of the subject. LI 18 point is a point located at a position resulting from shifting sideward by about 6 cm from the Adam's apple 60. More specifically, LI 18 point is a position where, when a horizontal line is drawn from the Adam's apple 60 along the neck circumference, the horizontal line and a sternocleidomastoid meet each other. Here, the sternocleidomastoid is a muscle that originates from the inside of the sternum and the clavicle and stops at the mastoid process.

FIG. 4B illustrates the back of the subject and illustrates a state in which the light irradiator 200 is mounted on each area corresponding to a vertebral artery of the cervix (neck) of the subject. For example, along the left and right sides of the midline 70, a straight-line distance from the midline 70 to a line vertically extending downward from an earlobe will be referred to as "back midline-earlobe distance" for convenience of description in the present disclosure. The back midline-earlobe distance may be about 1/4 of the neck circumference. Then, to mount the light irradiator 200 on the area corresponding to the vertebral artery, the light irradiator 200 may be mounted between a 1/2 point of the back midline-earlobe distance from the midline 70 and a 2/3 point of the back midline-earlobe distance from the midline 70.

Alternatively, when the light irradiator 200 is mounted on the area corresponding to the vertebral artery of the cervix (neck) of the subject as in FIG. 4B, the light irradiator 200 may be mounted on Urinary Bladder 10 (UB 10) point on the back of the neck (the back of the cervix) of the subject. Here, UB 10 point is an acupuncture point located at the back of the neck. UB 10 point is located at an outer border of each trapezius muscle located about 1 to 3 cm sideways from the midpoint of the posterior hairline (Governor Vessel 15 (GV 15) point, more specifically, a point located at a position resulting from shifting downward by about 1 cm from a position where the neck meets the skull at the middle of the back of the neck).

Alternatively, when the light irradiator 200 is mounted on the area corresponding to the vertebral artery of the cervix (neck) of the subject as in FIG. 4B, the light irradiator 200 may be mounted on Gallbladder 20 (GB 20) point on the back of the neck (the back of the cervix) of the subject. Here, GB 20 point refers to a depressed point located about 1.5 cm sideways from the center at the back of the neck on each of both sides of the neck. GB 20 point is located slightly more outward than UB 10 point.

In other words, the light irradiator 200 may be attached to areas corresponding to the left and right carotid arteries of the cervix of the subject in such a way that the light irradiator 200 is mounted between the 1/2 point of the front midline-earlobe distance from the midline 70 or the Adam's apple 60 and the 2/3 point of the front midline-earlobe distance from the midline 70 or the Adam's apple 60. Also, the light irradiator 200 may be attached to areas corresponding to the left and right vertebral arteries of the cervix of the subject in such a way that the light irradiator 200 is mounted between the 1/2 point of the back midline-earlobe distance from the midline 70 and the 2/3 point of the back midline-earlobe distance from the midline 70.

That is, two light irradiators 200 may be mounted on the areas corresponding to the carotid arteries of the cervix of the subject or mounted on the areas corresponding to the vertebral arteries of the cervix of the subject. According to circumstances, two light irradiators 200 may be mounted on the areas corresponding to the carotid arteries of the cervix and two light irradiators 200 may be mounted on the areas corresponding to the vertebral arteries of the cervix simultaneously.

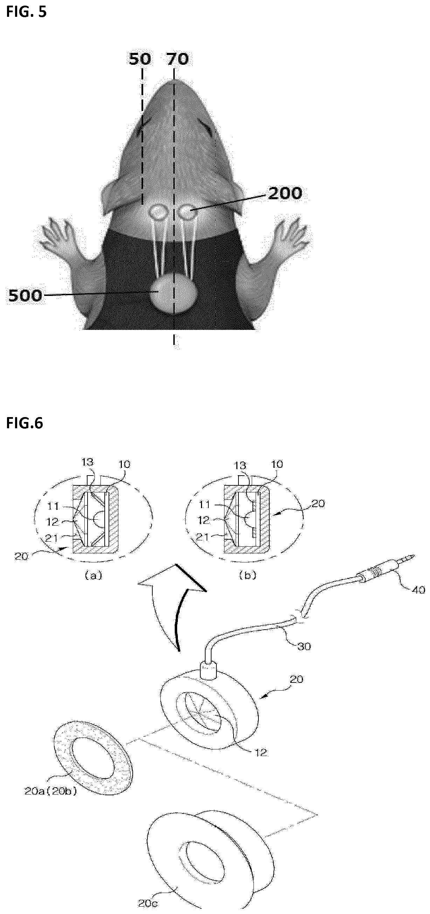

FIG. 5 is a view for describing positions at which the light irradiator according to the present disclosure is adhered or brought close to the cervix (neck) of an animal to irradiate visible light.

For animals (e.g., dogs, cats, and the like), in order to mount the light irradiator 200 on an area corresponding to a vertebral artery, the light irradiator 200 is adhered (mounted) or brought close to UB 10 point of the animal.

UB 10 point of animals (e.g., dogs, cats, and the like) may be defined as a point that is about halfway between the midline of the animal and an ear start point 5 at an upper portion of the head of the animal.

Also, although not illustrated, for animals (e.g., dogs, cats, and the like), in order to mount the light irradiator 200 on an area corresponding to a carotid artery, the light irradiator 200 is adhered (mounted) or brought close to LI 18 point of the animal.

LI 18 point of animals (e.g., dogs, cats, and the like) may be defined as a point that is about halfway between the midline of the animal and an ear end point of the animal.

A method for relaxing cerebral vessels using the device 100 for inducing improvement of cerebral circulation according to the present disclosure induces improvement of cerebral blood flow by a method in which, using the attachment tool 300, two light irradiators 200 are directly adhered or brought close to a skin surface located in each area corresponding to one of the left and right vertebral arteries of the occipital region of the human body or each area corresponding to one of the left and right carotid arteries of the neck to stimulate nerve endings, which are distributed in the dermis or epidermis located below the skin surfaces at the locations of the vertebral arteries or the carotid arteries, for 10 minutes to 20 minutes per time using the visible light emitted from the light irradiator 200 and induce secretion of a substance such as nitric oxide from nitrergic nerve terminals, which are connected to the stimulated nerve endings through the peripheral nerves, so that cerebral vessels which are in contact with the nitrergic nerve terminals are relaxed.

FIG. 6 illustrates, as an example of the light irradiator 200 according to the present disclosure, a cylindrical light irradiator using a single light source 11.

The cylindrical light irradiator includes a printed circuit board (PCB) 10 on which the light source 11 is installed, a body 20 which accommodates the PCB 10, and a power jack 40 connected to the body 20 through a wire 30.

The PCB 10 has the light source 11, which is one of an LED, an OLED, or a micro LED or a combination of one or more thereof, embedded therein and supplies driving power to the light source 11. According to circumstances, the driving power may be received from a battery embedded in the light irradiator 200 or may also be received from the light irradiation control device 500.

As illustrated in FIGS. 6A and 6B, which are enlarged views, a light filter 12 that is formed of plastic, glass, quartz, crystal, crystal glass or the like and filters and focuses light emitted from the light source 11 and emits the light through a light emission hole 21 is further installed on the PCB 10.

As illustrated in FIGS. 6A and 6B, which are enlarged views, a reflector 13 that is formed of a reflective plate, a reflective film, or the like and reflects colored light emitted from the light source 11 toward the light emission hole 21 is further installed around the light source 11.

The body 20 has the PCB 10 accommodated therein and has the light emission hole 21 formed in a front surface portion to intensively irradiate a treatment area of the animal or human body with therapeutic light. The body 20 has a cylindrical shape as illustrated in FIG. 6, but the present disclosure is not limited thereto. The shape of the body 20 may be changed to various shapes such as a patch shape, a hemispherical shape, a pot shape, an elliptical shape, a heart shape, and the like. The body 20 may be used in a state in which the body 20 is attached to an area of the animal or human body to be treated by colored light by using a double-sided adhesive tape 20a, a hydrogel pad 20b, an air suction plate 20c, or the like.

A wired, cylindrical light irradiator is illustrated in FIG. 6, but note that this is not intended to limit the present disclosure. Various other light irradiators such as a wireless light irradiator may also be adopted. In addition, light irradiators described in Korean Patent Publication No. 10-2012-0131316, Korean Patent Registration No. 10-1225135, Korean Patent Publication No. 10-2016-0049097, Korean Patent Publication No. 10-2016-0054423, etc., which have been previously disclosed by the inventors of the present disclosure, may also be applied.

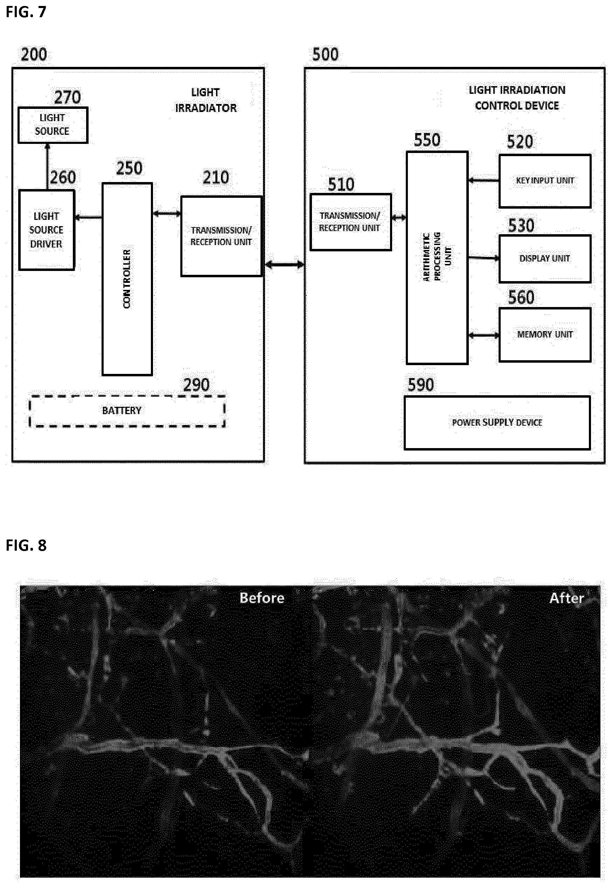

FIG. 7 is a block diagram illustrating a configuration of a device for inducing improvement of cerebral circulation according to the present disclosure.

The light irradiation control device 500 includes the display unit 530, the arithmetic processing unit 550, the wireless transmission/reception unit 510, a power supply device 590, a key input unit 520, and a memory unit 560.

Through the key input unit 520, driving of the light irradiator 200 may be started or ended, or setting data (treatment time, treatment intensity, treatment mode (one-time treatment or periodical treatment), and the like) may be set.

For example, when a light irradiation switch in the key input unit 520 is turned on, the arithmetic processing unit 550 outputs a light irradiation control signal and causes light to be emitted from the light irradiator 200 for a set light irradiation time and, when the set light irradiation time elapses, automatically outputs a light irradiation end signal and causes emission of the light from the light irradiator 200 to be stopped.

For example, when the light irradiation switch in the key input unit 520 is turned on by being pressed and held for three seconds or more or when a periodical treatment mode switch is selected (turned on), a predetermined program may cause light to be emitted, at every predetermined time interval (for example, every eight hours or every six hours), from the light irradiator for a set light irradiation time and, when the set light irradiation time elapses, automatically output a light irradiation end signal and cause emission of the light from the light irradiator to be stopped. In this way, light therapy may be automatically performed periodically on a pet or a patient who is unable to move his or her body.

The arithmetic processing unit 550 generates a light irradiation control signal according to the setting data (treatment time, treatment intensity, and the like) received from the key input part 520 and transmits the generated light irradiation control signal to the light irradiator 200 through the transmission/reception unit 510 of the light irradiation control device 500. Here, the transmission/reception unit 510 of the light irradiation control device 500 and a transmission/reception unit 210 of the light irradiator 200 may be wired transmission/reception units, which include a wired transmission/reception port, or wireless transmission/reception units.

Also, the arithmetic processing unit 550 stores the setting data (treatment time, treatment intensity, and the like) in the memory unit 560.

The power supply device 590 is a power supply device for power used when driving the light irradiation control device 500. According to circumstances, when the light irradiation control device 500 and the light irradiator 200 are connected by a wire, the light irradiator 200 may be driven by receive power supply from the power supply device 590 of the light irradiation control device 500. In this case, a battery 290 may be omitted.

The light irradiator 200 may include the transmission/reception unit 210, a controller 250, a light source driver 260, the light source 270, and the battery 290.

The controller 250 of the light irradiator 200 drives the light source driver 260 according to a light irradiation control signal received from the light irradiation control device 500 through the transmission/reception unit 210 of the light irradiator 200 and turns on or turns off the light source 270.

According to circumstances, the transmission/reception unit 210 of the light irradiator 200 may be mounted on the attachment tool 300, or the transmission/reception unit 210 and the controller 250 of the light irradiator 200 may be mounted on the attachment tool 300.

Two experiments were conducted to check performance of the device for inducing improvement of cerebral circulation according to the present disclosure. The two experiments and results thereof will be described below.

As the first experiment, the present inventors conducted an experiment to check whether cerebral vessels were relaxed and improvement of cerebral circulation was induced using the device and method for inducing improvement of cerebral circulation according to the present disclosure. An animal used in the experiment was a normal mouse (a C57BL6 mouse, age=20 week, n=5), the wavelength of light used was 610 nm, the intensity of the light was 1.4 mW/cm², and a light irradiation time was 20 minutes. To check relaxation of blood vessels and changes in the blood flow after light stimulation from the animal in vivo, the experimental animal was placed under a two-photon laser microscope, a light irradiation probe was attached to two points, each corresponding to one UB 10 point at the back of the neck, on the skin in a vertebral artery (VA) region from which hair was removed, and light stimulation was performed on the two points. To obtain microscopic images, the skull of the experimental animal was opened, the exposed cerebral cortex was covered by a transparent, flexible polydimethylsiloxane (PDMS) film, and then the cerebral cortex covered by the PDMS film was placed under an objective lens of the microscope to obtain cerebrovascular images before and after light stimulation.

FIG. 8 illustrates an example of a graph showing results of imaging cerebral vessels before and after light stimulation applied to an animal using a device and method for inducing improvement of cerebral circulation according to the present disclosure. The left image in FIG. 8 is a cerebrovascular image of the animal before the light stimulation, and the right image in FIG. 8 is a cerebrovascular image of the animal after the light stimulation.

As shown in FIG. 8, it was confirmed that cerebral vessels were relaxed and improvement of cerebral circulation was induced after the light stimulation.

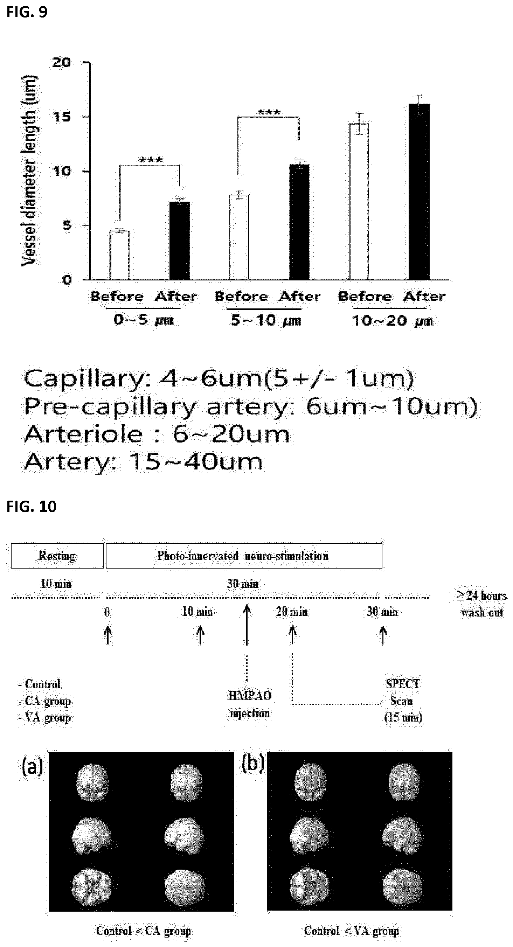

FIG. 9 illustrates graphs showing degrees of relaxation of blood vessels for each vessel diameter length before and after light stimulation experimented on an animal using the device and method for inducing improvement of cerebral circulation according to the present disclosure. That is, FIG. 9 shows results of measuring vessel diameter lengths before and after the stimulation, according to blood vessels having thicknesses in different ranges (0 to 5 µm, 5 to 10 µm, 10 to 20 µm).

As shown in FIG. 9, it was confirmed that relaxation of cerebral vessels occurred to a significant extent in blood vessels of all sizes such as a capillary, a pre-capillary artery, and an arteriole. From such a result, it was confirmed that the capillary was relaxed to a greater extent, particularly.

As the second experiment, the present inventors conducted an experiment to check, through single-photon emission computed tomography (SPECT) images, whether cerebral vessels were relaxed and improvement of cerebral circulation was induced in a normal person (n=10), by using the device and method for inducing improvement of cerebral circulation according to the present disclosure.

FIG. 10 illustrates an experimental protocol in which, using the device and method for inducing improvement of cerebral circulation according to the present disclosure, cerebral vessels are relaxed to induce improvement of cerebral circulation in a normal person and illustrates a result of conducting an experiment according to the experimental protocol.

In an imaging method using the protocol, SPECT imaging was performed for a total of three times on each subject. Here, SPECT imaging was re-performed 24 hours after previous SPECT imaging, in consideration of time taken for a contrast medium, which was administered for the previous SPECT imaging, to be washed out. The SPECT imaging performed three times was defined as Control, CA, or VA according to a stimulation method. Here, Control refers to a group without light stimulation, CA refers to a group in which an area corresponding to a carotid artery was stimulated, and VA refers to a group in which an area corresponding to a vertebral artery was stimulated.

The order of performing SPECT imaging was as follows. Before SPECT imaging, a subject rested for 10 minutes. The subject waited, lying on a table, and 15 minutes after the start (0 minute) of light stimulation, a contrast medium made of hexa methylene propylene amine oxime (HMPAO) was administered to the subject for SPECT imaging. The SPECT imaging was started 5 minutes after the contrast medium was administered. For about 15 minutes, 24 SPECT scans were obtained.

FIG. 10A shows results of the SPECT imaging expressed on 3D models by calculating a difference between a CA group and a Control group obtained from the same subject. Local blood flow changes were observed from parts of the frontal lobe and the occipital lobe of the brain.

FIG. 10B shows results of the SPECT imaging expressed on 3D models by calculating a difference between a VA group and the Control group. Blood flow changes were observed from the entire region of the brain.

The present disclosure may be applied to alleviating, treating, or preventing symptoms of various diseases, e.g., vascular dementia, Alzheimer's disease, Parkinson's disease, stroke, migraine, depression, and the like, which are closely related to cerebral circulation and improving memory and learning ability.

The present disclosure may be applied to alleviating, treating, or preventing symptoms of various diseases, e.g., dementia, Parkinson's disease, stroke, migraine, depression, and the like, which are closely related to cerebral circulation.

According to the device and method for inducing improvement of cerebral circulation according to the present disclosure, diseases caused by the lack of cerebral circulation can be treated or prevented by using photo-innervated neuro-stimulation (PINS) to stimulate, for a predetermined time, nerve endings distributed in the epidermis or dermis located below a skin surface in a specific region of a neck or a back portion of the neck, and inducing substances such as nitric oxide to be secreted from nitrergic nerve terminals connected to the stimulated nerve endings through the peripheral nervous system so that the secreted substances relax cerebral vessels in contact with the nitrergic nerve terminals and induce the improvement of cerebral circulation. Particularly, the device for inducing improvement of cerebral circulation according to the present disclosure do not require a cooling device, has a small size and weight, has low power consumption, is low-cost, and is easy to carry.

Also, the device and method for inducing improvement of cerebral circulation according to the present disclosure can be utilized in treating or preventing vascular dementia and Alzheimer's disease, which are caused by the lack of cerebral circulation, and various other brain-related diseases, such as stroke, migraine, depression, and Parkinson's disease, and improving learning ability, memory, and concentration. In this way, the present disclosure may significantly contribute to enhancement of the quality of life of the human race. The present disclosure may also significantly contribute to development of the industry of medical devices used to treat or prevent the above-mentioned various diseases.

The device and method for inducing improvement of cerebral circulation according to the present disclosure are not limited to the above-described embodiments. Various modifications may be made to the present disclosure by those of ordinary skill in the art without departing from the technical gist described in the appended claims, and such modifications also belong to the technical spirit of the present disclosure.

## Claims

1. A device for inducing improvement of cerebral circulation, the device comprising:
a light irradiator which includes a light source and irradiates, with light, an area corresponding to a carotid artery or an area corresponding to a vertebral artery, which is a skin surface of a cervix in an area where the carotid artery or the vertebral artery which supplies blood and oxygen to the brain is located, according to a light irradiation control signal; and
a light irradiation control device which generates the light irradiation control signal and transmits the light irradiation control signal to the light irradiator according to setting data including a light irradiation time.

2. The device of claim 1, wherein:
the light irradiator stimulates nerve endings distributed in the epidermis or dermis located below the skin surface in the area corresponding to the carotid artery or the area corresponding to the vertebral artery by irradiating the nerve endings with light for a predetermined light irradiation time and induces a substance containing nitric oxide to be secreted from nitrergic nerve terminals connected to the stimulated nerve endings through the nervous system; and
the secreted substance relaxes cerebral vessels and lymphatic vessels which are in contact with the nitrergic nerve terminals and induces the improvement of cerebral circulation.

3. The device of claim 2, wherein the light irradiator is controlled, by a light irradiation control device, to:
output visible light for suppressing accumulation of amyloid beta plaque by, as the cerebral vessels and the lymphatic vessels which are in contact with the nitrergic nerve terminals are relaxed and the improvement of cerebral circulation is induced, improving supply of oxygen and nutrients to cells, such as astrocytes, that are damaged which are responsible for functioning of the clearance system of the brain and gradually recovering impaired functions of the clearance system of the brain; and
emit the visible light for a set light irradiation time and automatically end emission of the visible light when the set light irradiation time ends.

4. The device of claim 1, wherein:
the light irradiation control device wirelessly transmits the light irradiation control signal to the light irradiator; and
the light irradiator has a battery embedded therein.

5. The device of claim 1, wherein:
the light irradiation control device transmits the light irradiation control signal to the light irradiator via a wire; and
the light irradiator is driven by power transmitted from the light irradiation control device.

6. The device of claim 1, wherein the area corresponding to the carotid artery is at a location of Large Intestine 18 (LI 18) point in the cervix of a human or an animal.

7. The device of claim 1, wherein the area corresponding to the vertebral artery is at a location of Urinary Bladder 10 (UB 10) point or Gallbladder 20 (GB 20) point in the cervix of a human or an animal.

8. The device of claim 1, wherein, when, along left and right sides of a midline or an Adam's apple at the front of the cervix, a straight-line distance from the midline or the Adam's apple to a line vertically extending downward from an earlobe is referred to as "front midline-earlobe distance," the light irradiator is mounted between a 1/2 point of the front midline-earlobe distance from the midline or the Adam's apple and a 2/3 point of the front midline-earlobe distance from the midline or the Adam's apple.

9. The device of claim 1, wherein, when, along left and right sides of a midline at the back of the cervix, a straight-line distance from the midline to a line vertically extending downward from an earlobe is referred to as "back midline-earlobe distance," the light irradiator is mounted between a 1/2 point of the back midline-earlobe distance from the midline and a 2/3 point of the back midline-earlobe distance from the midline.

10. The device of claim 1, wherein the light source is one of a light emitting diode (LED), an organic light emitting diode (OLED), or a micro LED or a combination of one or more thereof.

11. The device of claim 1, wherein the light irradiator is configured to emit light whose peak wavelength is in a range of 590 nm to 620 nm and whose intensity is in a range of 1 mW/cm² to 5 mW/cm².

12. The device of claim 1, wherein the light irradiator further includes an attachment tool which allows the light irradiator to be mounted on an animal or a human to irradiate the area corresponding to the carotid artery or the area corresponding to the vertebral artery with light.

13. The device of claim 12, wherein the attachment tool is one of a double-sided tape, a suction tool, a headgear, a necklace, and a neck strap.

14. The device of claim 1, wherein the light irradiator is configured to output light for 10 minutes to 20 minutes to stimulate the area corresponding to the carotid artery or the area corresponding to the vertebral artery one time.

15. The device of claim 1, wherein the light irradiation control device is one of a smartphone, a personal digital assistant (PDA), a laptop, an Android phone, an iPhone, a smartwatch, and a computer.

16. The device of claim 1, wherein the light irradiator further includes a light filter.

17. The device of claim 1, wherein, when a periodical treatment mode switch included in the light irradiation control device is selected, the light irradiation control device generates, at every predetermined time interval, a light irradiation control signal, which allows the light irradiator to output light for a predetermined light irradiation time, and transmits the light irradiation control signal to the light irradiator.

18. The device of claim 13, wherein, when the attachment tool is one of the headgear, the neck strap, and the necklace, a light irradiation control device or a light irradiator is embedded in the attachment tool.
